# EUROPÄISCHE PATENTSCHRIFT

(11) **EP 1 111 062 B1**
(45) Veröffentlichungstag und Bekanntmachung des Hinweises auf die Patenterteilung: **20.04.2005**
(21) Anmeldenummer: 00124042.3
(22) Anmeldetag: 04.11.2000
(51) Int. Cl.: C12N 15/77, C07K 14/34, C12P 13/08, C12N 15/31

(54) **Für das zwa1-Gen codierende Nukleotidsequenzen**
Nucleotide sequences encoding the gene zwa1
Séquences nucléotidiques codant pour le gène zwa1

(30) Priorität: 09.12.1999 DE 19959328
(43) Veröffentlichungstag der Anmeldung: 27.06.2001
(73) Patentinhaber: Degussa AG, 40474 Düsseldorf (DE)
(72) Erfinder: Möckel, Bettina, Dr., 40597 Düsseldorf (DE); Pfefferle, Walter, Dr., 33790 Halle (Westf.) (DE); Marx, Achim, Dr., 33613 Bielefeld (DE); Kalinowski, Jörn, Dr., 33615 Bielefeld (DE); Bathe, Brigitte, Dr., 33154 Salzkotten (DE); Pühler, Alfred, Prof., 33739 Bielefeld (DE)

(56) Entgegenhaltungen:
- EP-A- 0 435 132
- WO-A-01/04325
- KRAMER R: "Genetic and physiological approaches for the production of amino acids" JOURNAL OF BIOTECHNOLOGY, Bd. 45, Nr. 1, 12. Februar 1996 (1996-02-12), Seiten 1-21, XP004036833 AMSTERDAM NL ISSN: 0168-1656
- DATABASE EMBL [Online] Database accession no. L09232

## Beschreibung

Gegenstand der Erfindung sind die für das zwal-Gen kodierenden Nukleotidsequenzen und Verfahren zur fermentativen Herstellung von Aminosäuren, insbesondere L-Lysin, unter Verwendung von coryneformen Bakterien, in denen das zwal-Gen verstärkt wird.

### Stand der Technik

Aminosäuren, insbesondere L-Lysin, finden in der Humanmedizin und in der pharmazeutischen Industrie, insbesondere aber in der Tierernährung Anwendung.

Es ist bekannt, daß Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensbesserungen können fermentationstechnische Maßnahmen wie z. B. Rührung und Versorgung mit Sauerstoff, oder die Zusammensetzung der Nährmedien wie z. B. die Zuckerkonzentration während der Fermentation, oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Metabolite sind und L- Aminosäuren produzieren.

Seit einigen Jahren werden ebenfalls Methoden der rekombinanten DNA-Technik zur Stammverbesserung Aminosäureproduzierender Stämme von Corynebacterium eingesetzt, indem man einzelne Aminosäure-Biosynthesegene amplifiziert und die Auswirkung auf die Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al.(Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von Aminosäuren, insbesondere L-Lysin, bereitzustellen.

### Beschreibung der Erfindung

Aminosäuren, insbesondere L-Lysin, finden in der Humanmedizin, in der pharmazeutischen Industrie und insbesondere in der Tierernährung Anwendung. Es besteht daher ein allgemeines Interesse daran, neue verbesserte Verfahren zur Herstellung von Aminosäuren, insbesondere L-Lysin, bereitzustellen.

Wenn im folgenden L-Lysin oder Lysin erwähnt werden, sind damit nicht nur die Base, sondern auch die Salze wie z. B. Lysin-Monohydrochlorid oder Lysin-Sulfat gemeint.

Gegenstand der Erfindung ist ein isoliertes Polynukleotid aus coryneformen Bakterien, enthaltend eine Polynukleotidsequenz, ausgewählt aus der Gruppe
a) Polynukleotid, das zu mindestens 70 % identisch ist mit einem Polynukleotid, das für ein Polypeptid codiert, das die Aminosäuresequenz von SEQ ID NO 2 enthält,
b) Polynukleotid, das für ein Polypeptid codiert, das eine Aminosäuresequenz enthält, die zu mindestens 70% identisch ist mit der Aminosäuresequenz von SEQ ID NO 2,
c) Polynukleotid, das komplementär ist zu den Polynukleotiden von a) oder b), und
d) Polynukleotid, enthaltend mindestens 30 aufeinanderfolgende Nukleotide der Polynukleotidsequenz von a), b) oder c).

Gegenstand der Erfindung ist ebenfalls ein Polynukleotid gemäß Anspruch 1, wobei es sich bevorzugt um eine replizierbare DNA handelt, enthaltend:
(i) die Nukleotidsequenz, gezeigt in SEQ ID NO 1, oder
(ii) mindestens eine Sequenz, die den Sequenzen (i) innerhalb des Bereichs der Degeneration des genetischen Codes entspricht, oder
(iii) mindestens eine Sequenz, die mit zu den Sequenzen (i) oder (ii) komplementären Sequenzen hybridisiert, und gegebenenfalls

Weitere Gegenstände sind
ein Polynukleotid gemäß Anspruch 2, enthaltend die Nukleotidsequenzen wie in SEQ ID NO 1 dargestellt,wobei das Polynukleotid eine in coryneformen Bakterien replizierbare, bevorzugt rekombinante DNA ist,
ein Polynukleotid gemäß Anspruch 2, das für ein Polypeptid codiert, das die Aminosäuresequenz, wie in SEQ ID NO 2 dargestellt, enthält,
einen Vektor, enthaltend die für das zwal-Gen codierende DNA-Sequenz von C. glutamicum, enthalten in dem Vektor (Plasmid) pCR2.1zwalexp., hinterlegt in E. coli Top10F' unter der Nummer DSM 13115
und als Wirtszelle dienende coryneforme Bakterien, die den Vektor gemäß Anspruch 6 enthalten oder in denen das zwal-Gen verstärkt wird.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID NO 1 oder Teile davon enthält mit einer Sonde, die die Sequenz des genannten Polynukleotids gemäß SEQ ID NO 1 oder ein Fragment davon enthält und Isolierung der genannten DNA-Sequenz.

Polynukleotidsequenzen gemäß der Erfindung sind als Hybridisierungs-Sonden für RNA, cDNA und DNA geeignet, um cDNA in voller Länge zu isolieren, die für das Zwa1 Genprodukt codieren und um solche cDNA oder Gene zu isolieren, die eine hohe Ähnlichkeit der Sequenz mit der des zwa1-Gens aufweisen.

Polynukleotidsequenzen gemäß der Erfindung sind weiterhin als Primer geeignet, mit deren Hilfe durch die Polymerase-Kettenreaktion (PCR) DNA von Genen hergestellt werden kann, die für das zwal-Gen kodieren.

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden.

_{"}Isoliert" bedeutet aus seinem natürlichen Umfeld herausgetrennt.

_{"}Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter _{"}Polypeptiden" versteht man Peptide oder Proteine, die zwei oder mehr über Peptidbindungen verbundene Aminosäuren enthalten.

Die Polypeptide gemäß Erfindung schließen Polypeptide gemäß SEQ ID NO 2, insbesondere solche mit der biologischen Aktivität des Genproduktes des zwa1 Gens und auch solche ein, die zu wenigstens 70 % identisch sind mit dem Polypeptid gemäß SEQ ID NO 2, bevorzugt zu wenigstens 80% und besonders die zu wenigstens 90 % bis 95 % Identität mit dem Polypeptid gemäß SEQ ID NO 2 und die genannte Aktivität aufweisen.

Die Erfindung betrifft weiterhin ein Verfahren zur fermentativen Herstellung von Aminosäuren, insbesondere L-Lysin, unter Verwendung von coryneformen Bakterien, die insbesondere bereits die Aminosäure produzieren und in denen die für das zwal-Gen kodierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind zum Beispiel die bekannten Wildtypstämme
Corynebacterium glutamicum ATCC13032
Corynebacterium acetoglutamicum ATCC15806
Corynebacterium acetoacidophilum ATCC13870
Corynebacterium melassecoloa ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715

Den Erfindern gelang es, das neue, für das Zwal-Genprodukt codierende zwa1-Gen von C. glutamicum zu isolieren.

Zur Isolierung des zwa1-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αMCR, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z. B. bei Sanger et al. (Proceedings of the National Academy of Sciences of the United States of America, 74:5463-5467, 1977) beschrieben ist.

Auf diese Weise wurde die neue für das zwal-Gen kodierende DNA-Sequenz von C. glutamicum erhalten, die als SEQ ID NO 1 Bestandteil der vorliegenden Erfindung ist. Weiterhin wurde aus der vorliegenden DNA-Sequenz die Aminosäuresequenz des entsprechenden Genproduktes des zwa1-Gens abgeleitet. In SEQ ID NO 2 ist die sich ergebende Aminosäuresequenz des zwal-Genproduktes dargestellt.

Kodierende DNA-Sequenzen, die sich aus den SEQ ID NO 1 durch die Degeneriertheit des genetischen Codes ergeben, sind ebenfalls Bestandteil der Erfindung. In gleicher Weise sind DNA-Sequenzen, die mit der SEQ ID NO 1 oder Teilen von SEQ ID NO 1 hybridisieren, Bestandteil der Erfindung. In der Fachwelt sind weiterhin konservative Aminosäureaustausche wie z. B. ein Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben--Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2 ergeben, sind ebenfalls Bestandteil der Erfindung.

In gleicher Weise sind DNA-Sequenzen, die mit SEQ ID NO 1 oder Teilen von SEQ ID NO 1 hybridisieren, Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID NO 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Überexpression des zwa1-Gens in verbesserter Weise Aminosäuren, insbesondere L-Lysin, produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurde das erfindungsgemäße Gen zwa1 mit Hilfe der Integrationsmethode wie sie z. B. bei. Reinscheid et al. (Applied and Environmental Microbiology 60, 126-132 (1994)) beschrieben ist, dupliziert bzw. überexprimiert. Bei dieser Methode wird das vollständige Gen in einen Plasmidvektor kloniert, der in einem Wirt (typischerweise E. coli), nicht aber in C. glutamicum replizieren kann. Als Vektoren kommen bespielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-Patent 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm zwei Kopien des betreffenden Gens. Auf diese Weise wurde unter Zuhilfenahme des Integrationsplasmides pCR2.1zwa1exp der Stamm DSM5715:: pCR2.1zwa1exp hergestellt, der zwei Kopien des zwa1-Gens trägt.

Zusätzlich kann es für die Produktion von Aminosäuren insbesondere L-Lysin vorteilhaft sein, neben dem Zwa1-Genprodukt eines oder mehrere Enzyme des jeweiligen Biosyntheseweges der Glykolyse, der Anaplerotik, des Zitronensäure-Zyklus oder des Aminosäure-Exports zu verstärken, insbesondere zu überexprimieren.

So kann es vorteilhaft sein, beispielsweise für die Herstellung von L-Lysin, eines oder mehrere der Gene, ausgewählt aus der Gruppe
- das für die Dihydrodipicolinat-Synthase kodierende dapA-Gen (EP-B 0 197 335),
- das für eine feed back resistente Aspartatkinase kodierende lysC-Gen
- das für die Tetradihydrodipicolinat Succinylase kodierende dapD Gen (Wehrmann et al., Journal of Bacteriology 180, 3159-3165 (1998)),
- das Gen für die Succinyldiaminopimelate-Desuccinylase kodierende dapE Gen (Wehrmann et al., Journal of Bacteriology 177: 5991-5993 (1995)),
- das für die Glyceraldehyd-3-Phosphat Dehydrogenase kodierende gap-Gen (Eikmanns (1992). Journal of Bacteriology 174:6076-6086),
- das für die Pyruvat Carboxylase codierende pyc-Gen(Eikmanns (1992). Journal of Bacteriology 174:6076-6086),
- das für die Malat:Chinon Oxidoreduktase kodierende mqo-Gen (Molenaar et al., European Journal of Biochemistry 254, 395 - 403 (1998)),
- das für den ein Protein für Lysin-Export kodierende lysE-Gen (DE-A-195 48 222)
gleichzeitig zu überexprimieren.

Weiterhin kann es für die Produktion von Aminosäuren, insbesondere L-Lysin vorteilhaft sein, neben dem zwa1 Gen gleichzeitig
- das für die Phosphatpyruvat-Carboxykinase codierende Gen (DE 199 50 409; DSM 13047) und/oder
- das für die Glucose-6-Phosphat Isomerase codierende pgi-Gen (US 09/396,478; DSM 12969)
abzuschwächen.

Weiterhin kann es für die Produktion von Aminosäuren, insbesondere L-Lysin, vorteilhaft sein, neben der Überexpression des zwa1-Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) zum Zwecke der Produktion von Aminosäuren, insbesondere L-Lysin, kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an Lysin gebildet hat. Dieses Ziel wird normalerweise innerhalb von 10 Stunden bis 160 Stunden erreicht.

Die Analyse von L-Lysin kann durch Anionenaustauschchromatographie mit anschließender Ninhydrin Derivatisierung erfolgen, so wie bei Spackman et al. (Analytical Chemistry, 30, (1958), 1190) beschrieben.

Folgender Mikroorganismus wurde bei der Deutschen Sammlung für Mikrorganismen und Zellkulturen (DSMZ, Braunschweig, Deutschland) gemäß Budapester Vertrag hinterlegt:

Escherichia coli Stamm Top10F'/pCR2.1zwalexp als DSM 13115.

Das erfindungsgemäße Verfahren dient zur fermentativen Herstellung von Aminosäuren, insbesondere L-Lysin.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben, isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCosl (Wahl et al. (1987) Proceedings of the National Academy of Sciences USA 84:2160-2164), bezogen von der Firma Stratagene (La Jolla, USA, Produktbeschreibung SuperCosl Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A laboratory Manual, Cold Spring Harbor) beschrieben durchgeführt, wobei die Zellen auf LB-Agar (Lennox, 1955, Virology, 1:190) mit 100 µg/ml Ampicillin ausplattiert wurden. Nach Inkubation über Nacht bei 37°C wurden rekombinante Einzelklone selektioniert.

### Beispiel 2

### Isolierung und Sequenzierung des zwa1-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DHSαMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402) gegen die non-redundant Datenbank des "National Center for Biotechnology Information" (NCBI, Bethesda, MD, USA) durchgeführt.

Die erhaltene Nukleotidsequenz des zwa1-Gens ist in SEQ ID NO 1 dargelegt. Die Analyse der Nukleotidsequenz ergab ein offenes Leseraster von 597 Basenpaaren, welches als zwa1-Gen bezeichnet wurde. Das zwa1-Gen kodiert für ein Polypeptid von 199 Aminosäuren, welches in SEQ ID NO 2 dargelegt ist.

### Beispiel 3

### Herstellung eines Vektors zur Überexpression von zwa1

Aus dem Stamm ATCC 13032 wurde nach der Methode von Eikmanns et al. (Microbiology 140: 1817-1828 (1994)) chromosomale DNA isoliert. Aufgrund der aus Beispiel 2 für C. glutamicum bekannten Sequenz des zwa1-Gens wurden die folgenden Oligonukleotide für die Polymerase Kettenreaktion ausgewählt:
zwa1-d1:
   5' TCA CA CCG ATG ATT CAG GC 3'
zwa1-d2:
   5' AGA TTT AGC CGA CGA AAG CG 3'

Die dargestellten Primer wurden von der Firma MWG Biotech (Ebersberg, Deutschland) synthetisiert und nach der Standard-PCR-Methode von Innis et al. (PCR protocols. A guide to methods and applications, 1990, Academic Press) mit Pwo-Polymerase der Firma Boehringer die PCR Reaktion durchgeführt. Mit Hilfe der Polymerase-Kettenreaktion wurde ein ca. 1,0 kb großes DNA-Fragment isoliert, welches das zwal-Gen trägt.

Das amplifizierte DNA Fragment wurde mit dem TOPO TA Cloning Kit der Firma Invitrogen Corporation (Carlsbad, CA, USA; Katalog Nummer K4500-01) in den Vektor pCR2.1-TOPO (Mead at al. (1991) Bio/Technology 9:657-663) ligiert. Anschließend wurde der E. coli Stamm Top10F' mit dem Ligationsansatz (Hanahan, In: DNA cloning. A practical approach. Vol.I. IRL-Press, Oxford, Washington DC, USA) elektroporiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y.), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCR2.1zwa1exp genannt.

### Beispiel 4

### Verdoppelung des zwa1-Gens in dem Lysinproduzenten DSM 5715

Der in Beispiel 3 genannte Vektor pCR2.1zwa1exp wurde nach der Elektroporationsmethode von Tauch et al.(FEMS Microbiological Letters, 123:343-347 (1994)) in Corynebacterium glutamicum DSM 5715 elektroporiert. Bei dem Stamm DSM 5715 handelt es sich um einen AEC (Aminoethylcystein) resistenten Lysin-Produzenten. Der Vektor pCR2.1zwa1exp kann in DSM 5715 nicht selbständig replizieren und bleibt nur dann in der Zelle erhalten, wenn er ins Chromosom von DSM 5715 integriert hat. Die Selektion von Klonen mit ins Chromosom integriertem pCR2.1zwa1exp erfolgte durch Ausplattieren des Elektroporationsansatzes auf LB Agar (Sambrook et al., Molecular cloning: a laboratory manual. 2^{nd} Ed. Cold Spring Harbor Laboratory Press, Cold Spring Harbor, N.Y., 1989), der mit 15 mg/l Kanamycin supplementiert worden war. Für den Nachweis der Integration wurden nach der Standardmethode von Innis et al. (PCR protocols. A guide to methods and applications, 1990, Academic Press) mit Pwo-Polymerase der Firma Boehringer Kontroll-PCR Reaktionen durchgeführt. Durch Kombination der Primer zwa1-d1 und zwa1-d2 (vgl. Beispiel 3) mit den Primern M13 universal forward (5'gttttcccagtcacgac-3')(Invitrogen Corporation, Cat. No. N540-02) und M13 universal reverse (5'-caggaaacagctatgac-3')(Invitrogen Corporation, Cat. No. N530-02), die nur innerhalb der Sequenz des Vektors pCR2.1zwa1exp binden können, konnte gezeigt werden, daß das Plasmid pCR2.1zwa1exp in das Chromosom des Lysinproduzenten DSM5715 inseriert hatte. Der Stamm wurde als DSM5715::pCR2.1zwa1exp bezeichnet.

### Beispiel 5

### Herstellung von Lysin

Der in Beispiel 4 erhaltene C. glutamicum Stamm DSM5715::pCR2.1zwa1exp wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz Agar mit Kanamycin (25 mg/l) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Diesem wurde Kanamycin (25 mg/l) zugesetzt. Die Vorkultur wurde 48 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,1 betrug. Für die Hauptkultur wurde das Medium MM verwendet.

| Medium MM | |
|---|---|
| CSL (Corn Steep Liquor) | 5 g/l |
| MOPS | 20 g/l |
| Glucose(getrennt autoklaviert) | 50g/l |

| Salze: | |
|---|---|
| (NH₄)₂SO₄ | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 HO | 10 mg/l |
| MnSO₄ * H₂O | 5,0 mg/l |
| Biotin (sterilfiltriert) | 0,3 mg/l |
| Thiamin * HCl (sterilfiltriert) | 0,2 mg/l |
| Leucin (sterilfiltriert) | 0,1 g/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 48 Stunden wurde die OD bei einer Meßwellenlänge von 660 nm mit dem Biomek 1000 (Beckmann Instruments GmbH,

München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| DSM5715::pCR2.1zwa1exp | 12,1 | 11,93 |
| DSM5715 | 13,1 | 9,54 |

### SEQUENZPROTOKOLL

<110> Degussa-Hüls AG
   <120> Neue für das zwa1-Gen kodierende Nucleotidsequenzen
<130> 990172 BT
<140>
   <141>
<160> 2
<170> PatentIn Ver. 2.1
<210> 1
   <211> 1260
   <212> DNA
   <213> Corynebacterium glutamicum
<220>
   <221> -10_signal
   <222> (383)..(388)
<220>
   <221> -35_signal
   <222> (360)..(365)
<220>
   <221> CDS
   <222> (413)..(1009)
<400> 1
   ccgaaatatt ccaaatatgt aacataaatc acacccgatg attcaggcgg gatgacctgc 60
   gacttcaagg tcgcaccaaa gtcagattga tatagatttc gtaaataacg tgacacaatc 120
   gtgaccttcg ggttaccgtg tatcccaggc accgcaacag ttcatctgca agtccggctc 180
   atcgccaaac cctgtctggg gtcggaagtt gaacaacctc cttggtgcaa cagaacttta 240
   aaccacaaac tcccgcattc atgtgggcca tattgcagac agggacgggg aaaccaccca 300
   ccatcttttc acaaaagaag gcatggaggc caactccttg gggtgaagcc agacatccac 360
   tggcagagca actcctccgc tctaacccga cagctaacct cgacggcgac aa atg aga 418
                                                             Met Arg
                                                               1
   gga aaa ctt ttc atg gga cgt cac tcc act aag act agc tcc gcg ttc   466
   Gly Lys Leu Phe Met Gly Arg His Ser Thr Lys Thr Ser Ser Ala Phe
               5                  10                  15
   acc aag ctc gca gct tcc acc atc gct ttc ggt gct gct gca acc atc   514
   Thr Lys Leu Ala Ala Ser Thr Ile Ala Phe Gly Ala Ala Ala Thr Ile
        20                  25                  30
   atg gct cct tct gca tct gct gca cct gat tcc gac tgg gat cgc ctc   562
   Met Ala Pro Ser Ala Ser Ala Ala Pro Asp Ser Asp Trp Asp Arg Leu
    35                  40                  45                  50
   gca cag tgc gag tcc ggt ggt aac tgg gca atc aac acc ggt aac ggc    610
   Ala Gln Cys Glu Ser Gly Gly Asn Trp Ala Ile Asn Thr Gly Asn Gly
                   55                  60                  65
   tac cac ggt ggt ctg cag ttc tcc gct agc acc tgg gct gct tac ggc    658
   Tyr His Gly Gly Leu Gln Phe Ser Ala Ser Thr Trp Ala Ala Tyr Gly
               70                  75                  80
   ggc cag gag ttc gct acc tac gca tac cag gca acc cgt gag cag cag    706
   Gly Gln Glu Phe Ala Thr Tyr Ala Tyr Gln Ala Thr Arg Glu Gln Gln
           85                  90                  95
   atc gct gtt gca gag cgc acc ttg gct ggt cag ggc tgg ggc gca tgg    754
   Ile Ala Val Ala Glu Arg Thr Leu Ala Gly Gln Gly Trp Gly Ala Trp
       100                 105                 110
   cct gct tgc tcc gct tcc ctt gga ctg aac tcc gct cca acc cag cgt    802
   Pro Ala Cys Ser Ala Ser Leu Gly Leu Asn Ser Ala Pro Thr Gln Arg
   115                 120                 125                 130
   gac ctc tcc gct acc acc tcc acc cca gag cca gct gca gct gca cca    850
   Asp Leu Ser Ala Thr Thr Ser Thr Pro Glu Pro Ala Ala Ala Ala Pro
                   135                 140                 145
   gct gtt gct gag tac aac gct cct gca gcc aac atc gca gtt ggc tcc    898
   Ala Val Ala Glu Tyr Asn Ala Pro Ala Ala Asn Ile Ala Val Gly Ser
               150                 155                 160
   acc gac ttg aac acc atc aag tcc acc tac ggc gct gtc acc ggc acc    946
   Thr Asp Leu Asn Thr Ile Lys Ser Thr Tyr Gly Ala Val Thr Gly Thr
           165                 170                 175
   ctc gct cag tac ggc atc acc gtt cca gct gag gtt gag tct tac tac    994
   Leu Ala Gln Tyr Gly Ile Thr Val Pro Ala Glu Val Glu Ser Tyr Tyr
       180                 185                 190
   aac gct ttc gtc ggc taaatctagc tgcacttttt aaaagggagg gaaccttaaa   1049
   Asn Ala Phe Val Gly
   195
   cgggttccct ccctttttgc atgccatttc acgacgcgcc agtcatcctt ttgtgaattg 1109
   ggcaccaaga tttcctgatt ttggccacca ttttgccgaa accttggtgc cgaaagtacg 1169
   cccagtagaa aaaccgcatg aaaaaagagg caacaccgcc gaaacgggtt gcctcttttt 1229
   taagtttctt agcggttgat ccgggtgtac g                                1260
<210> 2
   <211> 199
   <212> PRT
   <213> Corynebacterium glutamicum
<400> 2

### Folgende Abbildungen sind beigefügt:

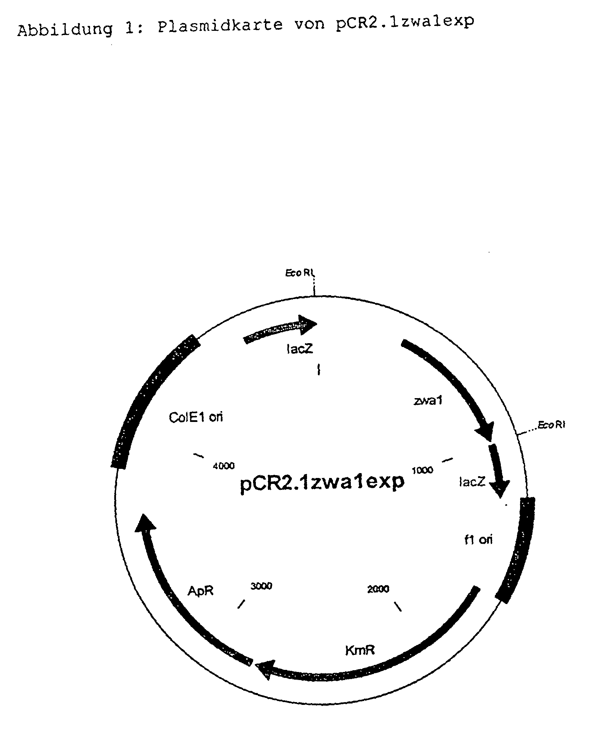

### Abbildung 1: Karte des Plasmids pCR2.1zwa1exp

Die Längenangaben sind als ca.-Werte zu verstehen.

Die verwendeten Abkürzungen und Bezeichnungen haben folgende Bedeutung.
- ColE1 ori:: Replikationsursprung des Plasmids ColE1
- lacZ:: 5'Ende des β-Galactosidase Gens
- f1 ori:: Replikationsursprung des Phagen f1
- KmR:: Kanamycin Resistenz
- ApR:: Ampicillin Resistenz
- EcoRI:: Schnittstelle des Restriktionsenzyms EcoRI
- zwa1: zwa1-Gen

## Patentansprüche

1. Isolierte Polynukleotide, die für ein Polypeptid mit der Aminosäuresequenz SEQ ID NO:2 kodieren.

2. Polynukleotide gemäss Anspruch 1, **gekennzeichnet durch** eine Nukleotidsequenz, ausgewählt aus der Gruppe:
a) Nukleotidsequenz gemäss SEQ ID NO:1, oder
b) eine Sequenz, die der Sequenz a)innerhalb des Bereichs der Degeneration des genetischen Codes entspricht, oder
c) Nukleotidsequenz mit den Positionen 413 bis 1009 gemäss SEQ ID NO:1.

3. Polynukleotid gemäss Anspruch 2 mit einer zu den Sequenzen a, oder b komplementären Sequenz.

4. Polynukleotide gemäss den Ansprüchen 1 bis 3 mit der Herkunft Corynebacterium.

5. In coryneformen Bakterien replizierbare Vektoren, die eine Nukleotidsequenz gemäss den Ansprüchen 1 bis 4 enthalten.

6. Coryneforme Bakterien, in denen die Expression eines Polynukleotids, das für ein Polypeptid mit der Aminosäuresequenz SEQ ID NO:2, in einem Umfang verstärkt wird, dass sich die L-Aminosäurenproduktion dieser Bakterien erhöht.

7. Coryneforme Bakterien gemäss Anspruch 6, in denen man die intrazelluläre Aktivität des Polypeptids mit der Sequenz SEQ ID NO:2 erhöht, indem man die Kopienzahl des für dieses Polypeptid kodierenden Polynukleotids erhöht oder einen starken Promoter verwendet und gegenenfalls diese Maßnahmen kombiniert.

8. Coryneforme Bakterien gemäss den Ansprüchen 6 oder 7, die mit einem Vektor, der ein für das Polypeptid mit SEQ ID NO:2 kodierendes Polynukleotid enthält, transformiert wurden.

9. Verfahren zur Herstellung von L-Aminosäuren, **dadurch gekennzeichnet, dass** man die Bakterien gemäss den Ansprüchen 6 bis 8 in einem geeigneten Medium fermentiert.

10. Verfahren gemäss Anspruch 9, **dadurch gekennzeichnet, dass** man
a) diese Bakterien in einem geeigneten Medium fermentiert,
b) die gewünschte(n) L-Aminosäure(n) im Medium oder in den Zellen der Bakterien anreichert, und
c) die L-Aminosäure(n) isoliert.

11. Verfahren gemäß den Ansprüchen 9 bis 11,
**dadurch gekennzeichnet,**
**dass** man coryneforme Bakterien verwendet, die L-Lysin herstellen.

12. Verfahren gemäß den Ansprüchen 9 bis 11,
**dadurch gekennzeichnet,**
**dass** man für die Herstellung von L-Lysin Bakterien fermentiert, in denen man gleichzeitig eines oder mehrere der Gene, ausgewählt aus der Gruppe
a) das für die Dihydrodipicolinat-Synthase kodierende dapA-Gen,
b) das für eine feed back resistente Aspartatkinase
c) das für die Tetradihydrodipicolinat Succinylase kodierende dapD-Gen,
d) das Gen für die Succinyldiaminopimelate-Desuccinylase kodierende dapE-Gen,
e) das für die Glyceraldehyd-3-Phosphat Dehydrogenase kodierende gap-Gen,
f) das für die Pyruvat-Carboxylase kodierende pyc-Gen,
g) das für die Malat:Chinon Oxidoreduktase kodierende mqo-Gen,
h) das für ein Protein für den Lysin-Export kodierende lysE-Gen,
verstärkt insbesondere überexprimiert oder amplifiziert.

13. Verfahren gemäß den Ansprüchen 9 bis 11,
**dadurch gekennzeichnet,**
**dass** man für die Herstellung von L-Lysin Bakterien fermentiert, in denen man
das für die Glucose-6-Phosphat Isomerase kodierende pgi-Gen
abschwächt.

14. Verfahren gemäß einem oder mehreren der vorhergehenden Ansprüche 9 bis 13,
**dadurch gekennzeichnet,**
**dass** man Mikroorganismen der Gattung Corynebacterium glutamicum einsetzt.

15. Vektor pCR2.1zwa1exp, hinterlegt unter der Bezeichnung DSM 13115 in E. coli.

16. Primer oder Sonde enthaltend mindestens 30 aufeinanderfolgende Nukleotide der Polynukleotidsequenz gemäss SEQ ID NO:1 enthält.

17. Primer oder Sonde gemäss Anspruch 16, **dadurch gekennzeichnet, dass** der Primer oder die Sonde mindestens 40 aufeinanderfolgende Nukleotid der Polynukleotidsequenz gemäss SEQ ID NO:1 enthält.

## Claims

1. Isolated polynucleotides, coding for a polypeptide having the amino acid sequence SEQ ID NO:2.

2. Polynucleotides according to Claim 1,
**characterized by** a nucleotide sequence selected from the group consisting of:
a) the nucleotide sequence according to SEQ ID NO:1, and
b) a sequence corresponding to the sequence a) within the range of the degeneracy of the genetic code, and
c) the nucleotide sequence with positions 413 to 1009 according to SEQ ID NO:1.

3. Polynucleotide according to Claim 2, having a sequence complementary to either of sequences a) and b).

4. Polynucleotides according to Claims 1 to 3, derived from Corynebacterium.

5. Vectors, which can be replicated in coryneform bacteria and which comprise a nucleotide sequence according to Claims 1 to 4.

6. Coryneform bacteria, in which expression of a polynucleotide for a polypeptide having the amino acid sequence SEQ ID NO:2 is enhanced to such an extent that production of L-amino acids in these bacteria increases.

7. Coryneform bacteria according to Claim 6, in which the intracellular activity of the polypeptide having the sequence SEQ ID NO:2 is increased by increasing the copy number of the polynucleotide coding for this polypeptide or by using a strong promoter and, where appropriate, combining these measures.

8. Coryneform bacteria according to Claim 6 or 7, which have been transformed with a vector comprising a polynucleotide coding for the polypeptide with SEQ ID NO:2.

9. Process for preparing L-amino acids, **characterized in that** the bacteria according to Claims 6 to 8 are fermented in a suitable medium.

10. Process according to Claim 9, **characterized in that**
a) the said bacteria are fermented in a suitable medium,
b) the desired L-amino acid(s) is (are) concentrated in the medium or in the bacterial cells, and
c) the L-amino acid(s) is (are) isolated.

11. Process according to Claims 9 to 11, **characterized in that** coryneform bacteria producing L-lysine are used.

12. Process according to Claims 9 to 11, **characterized in that** L-lysine is produced by fermenting bacteria in which simultaneously one or more of the genes selected from the group consisting of
a) the dapA gene, coding for dihydrodipicolinate synthase,
b) the lysC gene, coding for a feedback-resistant aspartate kinase,
c) the dapD gene, coding for tetradihydrodipicolinate succinylase,
d) the dapE gene, encoding the gene for succinyl-diaminopimelate desuccinylase,
e) the gap gene, coding for glyceraldehyde-3-phosphate dehydrogenase,
f) the pyc gene, coding for pyruvate carboxylase,
g) the mqo gene, coding for malate:quinone oxidoreductase,
h) the lysE gene, encoding a protein for lysine export,
are augmented, in particular overexpressed or amplified.

13. Process according to Claims 9 to 11, **characterized in that** L-lysine is produced by fermenting bacteria in which
the pgi gene, coding for glucose-6-phosphate isomerase,
is attenuated.

14. Process according to one or more of the preceding Claims 9 to 13, **characterized in that** microorganisms of the genus Corynebacterium glutamicum are used.

15. Vector, pCR2.1zwa1exp, deposited under the name DSM 13115 in E. coli.

16. Primer or probe, comprising at least 30 contiguous nucleotides of the polynucleotide sequence according to SEQ ID NO:1.

17. Primer or probe according to Claim 16, **characterized in that** the primer or the probe comprises at least 40 contiguous nucleotides of the polynucleotide sequence according to SEQ ID NO:1.

## Revendications

1. Polynucléotides isolés, qui codent pour un polypeptide avec la séquence d'acides aminés SEQ ID NO : 2.

2. Polynucléotides selon la revendication 1,
**caractérisés par**
une séquence nucléotidique, choisie parmi :
a) la séquence nucléotidique selon SEQ ID NO : 1, ou
b) une séquence, qui correspond à la séquence a) au sein d'un domaine de dégénérescence du code génétique, ou
c) une séquence nucléotidique avec les positions 413 à 1009 selon SEQ ID NO : 1.

3. Polynucléotide selon la revendication 2 avec une séquence complémentaire aux séquences a) ou b).

4. Polynucléotides selon l'une quelconque des revendications 1 à 3 provenant de Corynebacterium.

5. Vecteurs réplicables dans des bactéries corynéformes qui contiennent une séquence nucléotidique selon l'une quelconque des revendications 1 à 4.

6. Bactéries corynéformes,
**caractérisées en ce que**
l'expression d'un polynucléotide qui est renforcé pour un polypeptide avec une séquence d'acides aminés SEQ ID NO : 2, dans une quantité telle que la production d'acides L-aminés de ces bactéries augmente.

7. Bactéries corynéformes selon la revendication 6,
**caractérisées en ce qu'**
on augmente l'activité intracellulaire du polypeptide avec la séquence SEQ ID NO : 2, en augmentant le nombre de copies du polynucléotide codant pour ce polypeptide ou en utilisant un promoteur fort ou le cas échéant en combinant ces procédés.

8. Bactéries corynéformes selon l'une quelconque des revendications 6 ou 7, qui ont été transformées avec un vecteur qui contient un polynucléotide codant pour le polypeptide avec une SEQ ID NO : 2.

9. Procédé pour la fabrication d'acides L-aminés,
**caractérisé en ce qu'**
on fermente les bactéries selon l'une quelconque des revendications 6 à 8 dans un milieu approprié.

10. Procédé selon la revendication 9,
**caractérisé en ce qu'**
a) on fermente ces bactéries dans un milieu approprié,
b) on enrichit les acides L-aminés souhaités dans un milieu ou dans les cellules des bactéries, et
c) on isole les acides L-aminés.

11. Procédé selon l'une quelconque des revendications 9 à 11,
**caractérisé en ce qu'**
on utilise des bactéries corynéformes qui produisent de la L-lysine.

12. Procédé selon l'une quelconque des revendications 9 à 11,
**caractérisé en ce qu'**
on fermente pour la fabrication de L-lysine des bactéries, dans lesquelles on renforce, notamment on surexprime ou on amplifie parallèlement un des gènes ou plus choisis parmi :
a) le gène dapA, codant pour la dihydrodipicolinate synthase,
b) le gène lysC, codant pour une aspartate kinase résistante en retour,
c) le gène dapD, codant pour la tétradihydrodipicolinate succinylase,
d) le gène dapE, codant pour la succinyldiaminopimélate desuccinylase,
e) le gène gap, codant pour la glycéraldéhyde-3-phosphate déhydrogénase,
f) le gène pyc, codant pour la pyruvate carboxylase,
g) le gène mqo, codant pour la malate : quinone oxydoréductase,
h) le gène lysE, codant pour une protéine pour l'exportation de lysine.

13. Procédé selon l'une quelconque des revendications 9 à 11,
**caractérisé en ce que**
pour la production on fermente des bactéries de L-lysine, dans lesquelles on atténue le gène pgi, codant pour la glucose-6-phosphonate isomérase.

14. Procédé selon une quelconque ou plusieurs des revendications précédentes 9 à 13,
**caractérisé en ce qu'**
on utilise des micro-organismes du genre Corynebacterium glutamicum.

15. Vecteur pCR2.1zwa1exp, déposé sous la dénomination DSM 13115 dans E. Coli.

16. Amorce ou sonde contenant au moins 30 nucléotides successifs de la séquence polynucléotidique selon la SEQ ID NO : 1.

17. Amorce ou sonde selon la revendication 16,
**caractérisée en ce que**
l'amorce ou la sonde contient au moins 40 nucléotides successifs de la séquence polynucléotidique selon SEQ ID NO : 1.
